# EUROPEAN PATENT APPLICATION

(11) **EP 3 505 152 A1**
(43) Date of publication of application: **03.07.2019**
(21) Application number: 18745141.4
(22) Date of filing: 23.01.2018
(51) Int. Cl.: A61K 8/81, A61K 8/19, A61K 8/34, A61Q 5/10

(54) **HAIR DYE COMPOSITION, AND PREPARATION METHOD AND APPLICATION METHOD THEREOF**

(30) Priority: 26.01.2017 CN 201710056868
(71) Applicant: Shanghai Clinical Engine Technology Development Co., Ltd., Shanghai 200439 (CN)
(72) Inventor: SUN, Yinghao, Shanghai 200433 (CN)
(74) Representative: Viering, Jentschura & Partner mbB Patent- und Rechtsanwälte
(86) International application number: PCT/CN2018/073691
(87) International publication number: WO 2018/137587

(57) **Abstract**

A hair dye composition preparation method and application method thereof. The hair dye composition comprises a pigment, a pigment solubility enhancement carrier, and an arbitrary additive. The application method of the hair dye composition comprises: applying hair dye on dry or semi-wet hair having various colors; and washing off after 3-15 minutes to complete the hair dyeing procedure. The composition has simple components, has favorable color depth and color tone, and requires short hair dyeing time, achieving a controllable hair dyeing process.

## Description

### TECHNICAL FIELD

This invention relates to compositions. In particular, the invention relates to the composition, and preparation method, application method and use thereof. More specifically, the present invention relates to non-chemical oxidative hair dye, preparation method and application method thereof. The present invention relates to the fields of fine chemical industry and nanometer materials technology.

### BACKGROUND

At present, conventional hair dyeing is a cosmetic, the component of which penetrates deep into the interior of the hair, and adhering onto the hair surface with a small amount by destroying the chemical composition of the hair and coloring by chemical reaction, so as to achieve the purpose of hairdressing. The composition is mainly classified into two major categories, an oxidized composition and a direct type composition, depending on the chemical nature of the product. According to its dyeing effect, it can be distinguished as permanent, semi-permanent and temporary composition.

Most of the current market uses permanent hair dye, permanent hair dye includes natural dyes, metallic dyes and organic synthetic dyes, as shown in the following table:

Listed below are some oxidative dye raw materials and their dyeable colors.

| Dye developer | Colour | Dye couplant name | Colour |
|---|---|---|---|
| P-phenylenediamine | Brownish black | Quinol | Brown |
| P-toluenediamine | Brown | Resorcinol | Navy blue |
| P-aminodiphenylamine | Dark gray | P-aminophenol | Golden brown |
| P-phenylenediamine chloride | Dark brown | M-aminophenol | Dark gray purple |
| M-phenylenediamine | Purple | O-aminophenol | Gold |
| M-toluenediamine | Purple | 1,2,3-benzenetriol | Gold |
| O-phenylenediamine | Golden | α-naphthol | Light purple |
| O-toluenediamine | Light brown | Resorcinol | Brownish black |

At present, the dyeing mechanism of these dyes is first open the hair by using alkali agent, so that the oxidation dye enters the hair cortex layer, and then the hydrogen peroxide enters to react with the dyes, and the generated polymer material is directly deposited on the hair surface, thus achieving the goal of permanent hair dyeing. It is reported that such chemical compositions have been exposed to different degrees of carcinogenic cases, and the long-term use and high toxicity both can gradually cause mild or severe allergic reactions to the skin. The alkali agent used therein has obvious odor and is highly irritating. The erosion of hydrogen peroxide into hair causes the mechanical properties of the hair to decrease, the microstructure changes, and the hair is brittle and fragile.

As mentioned above, the current chemical dyeing process is complicated and causes great damage to hair; moreover, the current hair dyeing process takes long time, generally about 2 hours; due to the complex composition of the hair dye, it can only be used by professionals, and is not convenient. Therefore, it is expected to develop a new hair dye that has simple components, pure natural property, no irritating, no allergenicity, no mutagenicity, no odor, no damage on hair, short dyeing time, and family dyeing.

At the same time, with the deepening of research on nanotechnology and nanomaterials, people have also tried to synthesize ultrafine polymer particles (10-100nm) by polymer synthesizing method. Some research groups prepared nano-polymers by changing the polymer structure and the composition of the reaction system, in which the soap-free emulsion polymerization method has the most prominent application prospect in synthesizing biomedical polymer water-soluble pigment solubilizing carrier. Pigment molecules may be loaded on natural non-toxic nanocarriers for promoting their binding to hair, which will undoubtedly overcome the adverse effects of traditional hair dyeing techniques.

Therefore, for natural non-toxic hair dye molecules, there is a need for compositions that are fast, convenient, efficient, and non-toxic.

### SUMMARY

From the viewpoint of biomedical applications, the inventors of the present invention have synthesized a polymer having a certain composition by chemical synthesis and polymer polymerization method, directing at the disadvantages of high toxicity, long time, low efficiency, and poor hair dyeing effect in the dyeing of traditional chemical hair dyes and plants, wherein the polymer is a functional carrier capable of loading a large amount of natural non-toxic pigment. Massive loading of the pigment is achieved by the carrier, and its binding to the hair is enhanced without changing the structure and properties of the hair. A non-toxic, natural, fast, convenient and efficient composition is obtained by a certain ratio to achieve fast, convenient, non-toxic and efficient hair dyes and their applications so as to solve the problems existing in current hair dyeing technology.

The present invention provides compositions, preparation methods, application methods and use thereof.

In a first aspect of the invention, there is provided a composition comprising: pigments, water soluble pigment solubilizing carriers, and optional additives; Preferably, the water-soluble solubilizing carrier is 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27 %, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50% by mass in the composition and the range between the above respective values, including but not limited to 1-50%, preferably 1-40%, more preferably 1-30%, and most preferably 1-20%; and the pigment is 1%, 2%, 3%, 4%, 5%, 6%, 7%, 8%, 9%, 10%, 11%, 12 %, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40% by mass in the composition and the range between the above respective values, including but not limited to 1- 40%, preferably 1-30%, more preferably 1-20%, and most preferably 1-10%. The ratio is the weight ratio based on the entire composition. The composition is applicable to hair dyeing.

In a second aspect of the present invention, there is provided a method of dyeing hair by a composition including the steps of: contacting hairs with a composition of the invention containing a pigment, a water-soluble pigment solubilizing carrier, and optional additives as described above at 25°C to 50°C (preferably 50°C); the pigment is carbon black, graphene and a blend thereof, and the concentration of the carbon black solution is about 5-50 mg/mL; or the pigment is a pigment molecule (basic red, basic yellow, basic blue) and an basic black pigment obtained by formulating them according to a certain weight ratio, and the basic black pigment is formulated according to basic red : basic yellow : basic blue of 0.01-0.3:0.7-5:0.4-4 by weight ratio; or the pigment is a natural plant pigment molecule (carmine, lemon yellow, bright blue) and a natural plant black pigment obtained by formulating them according to a certain weight ratio, and the black pigment of the natural plant pigment is formulated according to carmine: lemon yellow: bright blue of 0.1-3:0.03-2:0.1-10 by weight ratio; wherein the pigment is a particle range from 0.1-5000 nm, preferably range from 0.1-500 nm. Further, the composition further includes deionized water. Herein, the hair is dyed by the pigment; after 3-15 min (3-10 min, preferably 5-10 min), rinsed with water and drained to obtain the hair to the desired color; then dyed by hair dye; rinsed with water and drained to obtain the hair dyed with different colors.

The interaction of the hair with the pigment and the water-soluble pigment in the composition of the present invention achieves a non-invasive and non-contaminating hair dyeing purpose. Specifically, the composition is added to the hair cortex layer by a water-soluble pigment solubilizing carrier which load pigment. With the bonding between the water-soluble pigment solubilizing carrier and the surface of the hair, the pigment is more firmly bonded to the hair to achieve permanent hair dyeing.

At the same time, the dyeing method of the present invention has the following characteristics: a water-soluble pigment solubilizing carrier and an optional alcohol (such as, glycerin) and the like, so that the composition of the present invention can quickly interact with the hair after dyeing the hair, take rapid complexation reaction, has short hair dyeing time (less than 10 min, preferably 3-10 min), and form a strong complex and allowing the pigment-loading solubilizing carrier to be quickly enclosed in the hair, so as to fastly achieve the effect of dyeing the hair.

A third aspect of the invention relates to the use of the composition in the preparation of hair dye.

A fourth aspect of the invention relates to a method of preparing the composition, including the step of mixing each component of the composition.

### BRIEF DESCRIPTION OF THE DRAWINGS

FIG. 1. illustrates comparison of dyeing results before and after dip-dyeing of ink brush hair instead of real hair, wherein before dip-dyeing (left) and after dip-dyeing (right).
FIG. 2. illustrates comparison of primary dyeing of different formulations before and after dip-dyeing, wherein before dip-dyeing (left) and after dip-dyeing (right).
FIG. 3 illustrates repeated experiment with rabbit hair instead of ink brush hair.
FIG. 4 illustrates comparison of dyeing conditions at different temperature and pH.
FIG. 5 illustrates comparison of the effects of different pH on hair dyeing.
FIG. 6 illustrates comparison of the effects of different times on hair dyeing.
FIG. 7 illustrates comparison of hair microstructure before and after hair dyeing.
FIG. 8 illustrates comparison of the effect of dyeing white rabbit hair with water-soluble pigment solubilizing carrier loading different dye.
FIG. 9 illustrates comparison of the effect of different water-soluble pigment solubilizing carrier loaded black pigments on white hair.
FIG. 10 illustrates comparison of the effect of the dyeing of different dyes on black human hair by water-soluble pigment solubilizing carrier in simulation of commercially available hair dyeing processes.
FIG. 11 illustrates comparison of dyeing of different dyes on black human hair by water-soluble pigment solubilizing carrier.
FIG. 12 illustrates study on the comparison of dye adhesion by water-soluble pigment solubilizing carrier.
FIG. 13 illustrates comparison of the effects of water-soluble pigment solubilizing carriers on hair quality after dyeing.
FIG. 14 illustrates comparison of evaluation for injury of epithelial cells by different components.
FIG. 15 illustrates comparison of dyeing effect evaluation of water-soluble pigment solubilizing carrier and commercially available hair dye.
FIG. 16 illustrates comparison of the rapid dyeing evaluation of water-soluble pigment solubilizing carriers effecting on commercially available dyeing paste.
FIG. 17 illustrates comparison of rapid dyeing evaluation of black dyeing paste based on water-soluble pigment solubilizing carrier.
FIG. 18 illustrates comparison of rapid dyeing evaluation of color dyeing paste based on water-soluble pigment solubilizing carrier.

### DEFINATION

As used herein, the terms "hair" and "hair" refer to any keratin-containing fiber or fiber material, including any human hair, animal hair (including but not limited to, rabbit hair) and ink brush hair.

As used herein, the term "pigment" refers to a fine, insoluble, white, black or colored substance that is used to render the pigment composition colored and/or opaque. The pigments are carbon black, graphene and blends thereof; or the pigment is a pigment molecule (basic red, basic yellow, basic blue) and a black pigment obtained by formulating them according to a certain weight ratio, and the black pigment is formulated according to basic red : basic yellow : basic blue of 0.01-0.3:0.7-5:0.4-4 by weight ratio; or the pigment is a natural plant pigment molecule (carmine, lemon yellow, bright blue) and a natural plant black pigment obtained by formulating them according to a certain weight ratio, and the black pigment of the natural plant pigment is formulated according to carmine : lemon yellow : bright blue of 0.1-3:0.03-2:0.1-10 by weight ratio; the pigment is a particle range from 0.1-5000 nm, preferably range from 0.1-500 nm.

As used herein, the standard for excellent hair dyeing effect is the objective evaluation criterion for the dyeing effect of hair according to dyeing time, dyeing amount and dyeing luster after visual inspection of hair dyeing effect by the person not involved in the experiment: poor (score 0-1), general (score 1-2), good (score 2-3), very good (score 3-4), and excellent (score 4-5).

As used herein, the term "water-soluble pigment solubilizing carrier" refers to any carrier that is capable of bonding to the surface of the hair and that is capable of enhancing the loading of the pigment and enhancing the softness of the hair surface layer.

As used herein, other colors may be formulated in a ratio of red, yellow and blue primary colors, for example, black is formulated by basic red : basic yellow : basic blue=0.01-0.3:0.7-5:0.4-4 (weight ratio); and carmine is formulated by lemon yellow : bright blue=0.1-3:0.03-2:0.1-10 (weight ratio).

As used herein, the term "room temperature" means 25±1°C. At the same time, if the experimental temperature is not specified, it is room temperature.

As used herein, the term "about" refers to ±20%, preferably ±10%, more preferably ±5% of the value modified by the term, so that ordinary skill in the art will be able to clearly modify Numerical values determine the scope of the term "about."

A first aspect of the invention relates to composition including: (1) pigments is 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29 %, 30%, 31%, 32%, 33%, 34%, 35%, 36%, 37%, 38%, 39%, 40% by mass in the composition and range between the above respective values thereof, including but not limited to 1-40%, preferably 1 -30%, more preferably 1-20%, and most preferably 1-10%; the pigment is carbon black, graphene or blends thereof, or the pigment is a pigment molecule (basic red, basic yellow, basic blue) and a black pigment obtained by formulating them according to a certain weight ratio, the basic black pigment is formulated according to basic red: basic yellow : basic blue, respectively, of 0.01-0.3:0.7-5:0.4-4 by weight ratio; or the pigment is a natural plant pigment molecule (carmine, lemon yellow, bright blue) and a natural plant black pigment obtained by formulating them according to a certain weight ratio, and the black pigment of the natural plant pigment is formulated according to carmine red : lemon yellow : bright blue of 0.1-3:0.03-2:0.1-10 by weight ratio; the pigment is a particle range from 0.1 and 5000 nm, preferably range from 0.1 and 500 nm; (2) water-soluble pigment solubilizing carrier, wherein the water-soluble pigment solubilizing carrier is 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40% 41%, 42%, 43%, 44%, 45%, 46%, 47%, 48%, 49%, 50% by mass in the composition, and the range between the above respective values thereof, including but not limited to 1-50%, preferably 1-40%, more preferably 1-30%, and most preferably 1-20%; further, the water-soluble pigment solubilizing carrier is selected from polymer, copolymer (the copolymer is prepared by the following method: free radical initiator initiates first monomer and second monomer both of which containing amino groups by a radical graft copolymerization reaction to form a copolymer in an aqueous solution at temperature of 60-100°C.) or inorganic material, wherein the polymer is a polymer containing carboxyl group, amino group, cationic group, and anionic group, and the inorganic material is an inorganic molecule containing silicon and oxygen elements; preferably, the polymer is a polymer selected from polyethyleneimine, polylysine, chitosan, sodium alginate, and polyacrylic acid; and polymers and copolymers thereof, and hybrid and composite material of the copolymer of the polymer and inorganic materials selected from silicon or iron; more preferably, the polymer is a polymer selected from polyethyleneimine, polylysine, chitosan, sodium alginate, polyacrylic acid, and the copolymer of the above-mentioned polymer linked with poly-isopropyl acrylamide or polymethyl methacrylate; preferably, the water-soluble pigment solubilizing carrier is a copolymer selected from polyethyleneimine-co-isopropylacrylamide, sodium alginate-co-polyethyleneimine, polyethyleneimine-co-isopropylacrylamide, chitosan-co-isopropylacrylamide, chitosan chain, and a nanocomposite system formed by non-chemical crosslinking; further, the water-soluble pigment solubilizing carrier includes forms of gel, latex, inorganic-organic hybrid composite, and self-assembled microsphere or nanogel; (3) additives selected from a derivative of glycerol/or salt or ester thereof, or a derivative of tridecyl polyether-2/or salt or ester thereof, and pentasodium pentoxide/or Tetrasodium pyrophosphate/or sodium stannate; the additive has a mass percentage of 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29 %, 30%, 31%, 32%, 33%, 34%, 35%, 36%, 37%, 38%, 39%, 40% in the composition, and range between the respective values thereof, including but not limited to 1-40 %, preferably 1-30%, more preferably 1-20%, and most preferably 1-10%. The composition further includes water, and the pH of the composition is adjusted to less than about 13 by the basic agent, specifically, a pH of 8, 9, 10, 11, 12, and range between the respective pH, including but not limited to:8<pH<13, preferably 9<pH<12, more preferably 8<pH<10, wherein the basic agent is alkanolamine and inorganic compound; the alkanolamine is a derivative of monoethanolamine, polyethanolamine or propylene glycol; the inorganic compound is sodium hydroxide, sodium carbonate, sodium hydrogencarbonate, calcium carbonate or calcium hydrogencarbonate. Preferably, the composition is solution, paste or emulsion.

The compositions of the present invention can be used to dye hair.

A second aspect of the invention relates to a method of applying the composition in dyeing hair, the method including the steps of: at 25°C-50°C (preferably 50°C), contacting the hair with a composition of the invention including pigment, water-soluble pigment solubilizing carrier and optional additives as described above, wherein the pigment is a particle range from 0.1-5000 nm, preferably range from 0.1-500 nm; The pigment is carbon black, graphene and mixture thereof, and the concentration of the carbon black solution is about 5-50 mg/mL; or the pigment is a pigment molecule (basic red, basic yellow, basic blue) and a black pigment obtained by formulating them in a certain weight ratio, and the basic black pigment is formulated according to basic red : basic yellow : basic blue of 0.01-0.3:0.7-5:0.4-4 by weight ratio; or the pigment is a natural plant pigment molecule (carmine, lemon yellow, bright blue) and a natural plant black pigment obtained by formulating them in a certain weight ratio. The black pigment of the natural plant pigment is formulated according to carmine red : lemon yellow : bright blue of 0.1-3:0.03-2:0.1-10 by weight ratio; further, the composition further includes deionized water. Herein, the hair is dyed by the pigment; after 3-15 min (3-10 min, preferably 5-10 min), rinsed with water and drained to obtain the hair of the desired color.

A third aspect of the invention relates to the use of the composition for the preparation of hair dye.

A fourth aspect of the invention relates to a method for preparing the composition including the step of mixing each component of the composition.

A method for preparing copolymer includes the free radical initiator initiates the first monomer and the second monomer both of which containing amino groups to form a copolymer by free radical graft copolymerization in an aqueous solution and at temperature of 60-100°C.

### DETAILED DESCRIPTION OF THE INVENTION

The present invention is further illustrated in combination with the accompanying drawings and the specific embodiments below hereinafter. It is to be understood that the following embodiments are merely illustrative of the invention and are not intended to limit the scope covered by the claims of the present invention.

The methods in the following embodiments, unless otherwise stated, are conventional methods.

### Embodiment 1: Effects on carbon black dyeing with or without water-soluble pigment solubilizing carrier

Experimental procedure: A small amount of ink brush hair was taken and burned with fire, producing a protein burning smell, and the hair was discriminated as true vellus and can be used. 100 mg hydrophilic carbon black was weighed into a tube, and 20 mL water (i.e., distilled water, the same below) was added, and the mixture was thoroughly stirred for 2 hours to prepare 5 mg/mL carbon black aqueous solution for use. Two equal volumes (1 mL, if not explicitly stated, the volumes are used in the following embodiments) of carbon black aqueous solution were taken respectively and labeled as No. 1 and No. 2. 1 mL water was added to the No. 1 tube, and 1 mL polyethyleneimine-co-isopropylacrylamide at a concentration of 4 mg/mL (if not explicitly stated, the concentration and volume were used in the following embodiments) was added to the No. 2 tube (water-soluble pigment solubilizing carrier), which both were fully oscillated to make it evenly mixed. At room temperature, two brushes were taken to dip-dye in No. 1 and No. 2 tubes respectively. Photos were taken to store before dyeing, and after dyeing, photos were taken to store after drying at room temperature.

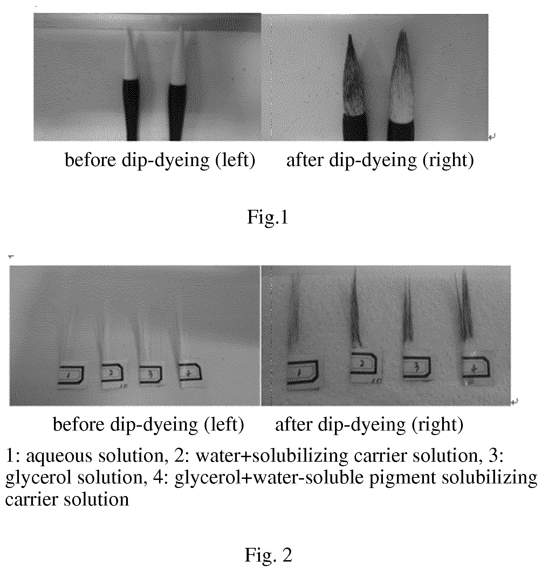

Experimental results: see the left figure of FIG. 1 (the left side of the figure without adding water-soluble pigment solubilizing carrier is No. 1 and the right side with the water-soluble pigment solubilizing carrier added is No. 2) and the right side of FIG. 1 (the ink brush on the left side of the figure is dyed with carbon black aqueous solution after adding water-soluble pigment solubilizing carrier, and the ink brush on the right side is dyed with carbon black aqueous solution without adding water-soluble pigment solubilizing carrier solution).

As can be seen from FIG. 1, under the same experimental conditions, the dyeing of the ink brush hair after adding water-soluble pigment solubilizing carrier had better dyeing effect compared to the ink brush hair without adding solubilizing carrier (the dyeing effects score were 2.5 and 0.5 respectively).

### Embodiment 2: Effect on carbon black dyeing with or without water-soluble pigment solubilizing carrier and/or glycerol

Experimental procedure: as in Embodiment 1, 20 mL carbon black aqueous solution having a concentration of 5 mg/mL was prepared, and 20 mL glycerol-carbon black solution was additionally prepared for use (the dissolution of carbon black in 20% glycerol took a certain time, with the magnetic rotor added to stir thoroughly to dissolve completely); two equal volumes of aqueous carbon black solution were labeled as No. 1 and No. 2 respectively, and two equal volumes of 2 ml glycerol-carbon black solution were labeled as No. 3 and No. 4.

1 mL water was added to the No. 1 tube, add 1 mL polyethyleneimine-co-isopropylacrylamide (water-soluble pigment solubilizing carrier) at a concentration of 4 mg/mL to the No. 2 tube, 1 mL glycerol to the No. 3 tube, and 1 mL polyethyleneimine-co-isopropylacrylamide (water-soluble pigment solubilizing carrier) at a concentration of 4 mg/mL and 1 mL glycerol to the No. 4 tube. The equal volumes of ink brush hair was taken and stuck with label paper and marked No. 1, 2, 3 and 4. At room temperature, dip-dyeing was carried out in 4 tubes for 5 min, and dried to observe the color change.

Experimental results: after the dip-dyeing, it can be seen from the right figure that the ink brush of No. 1 aqueous solution was dyed lighter and had poor effect compared to the No. 2 water-soluble pigment solubilizing carrier ink brush; the ink brush dyeing of the No. 1 aqueous solution was compared with the No. 3 glycerin solution, the No. 3 glycerin solution was dyed deeper without particle adhesion, the surface of the hair was smoother and the effect was better; however, on the whole, the No. 4 water-soluble pigment solubilizing carrier-water and glycerin had the best ink brush dyeing effect.

### Embodiment 3: Effect on carbon black dyeing with or without carbon-soluble pigment solubilizing carrier and/or glycerol (repeating Embodiment 2 with rabbit hair instead of ink brush)

Experimental procedure: as in Embodiment 2, 20 mL carbon black aqueous solution having a concentration of 5 mg/mL was prepared, and 20 mL glycerol-carbon black solution was additionally prepared for use (the dissolution of carbon black in glycerol took a certain time, with the magnetic rotor added to stir thoroughly to dissolve completely), two equal volumes of aqueous carbon black solution (1 ml) were labeled as No. 1 and No. 2 respectively, and two equal volumes of 5 ml glycerol-carbon black solution were labeled as No. 3 and No. 4. 1 mL water was added to the No. 1 tube, 1 mL polyethyleneimine-co-isopropylacrylamide (water-soluble pigment solubilizing carrier) at a concentration of 4 mg/mL to the No. 2 tube, 1 mL glycerol to the No. 3 tube, and 1 mL polyethyleneimine-co-isopropylacrylamide (water-soluble pigment solubilizing carrier) and 1 mL glycerol to the No. 4 tube. 10 mg rabbit hair was taken and glued with label paper and marked No. 1, 2, 3 and 4. At room temperature, dip-dyeing was carried out in 4 tubes for 10-30 min, and dried to observe the color change.

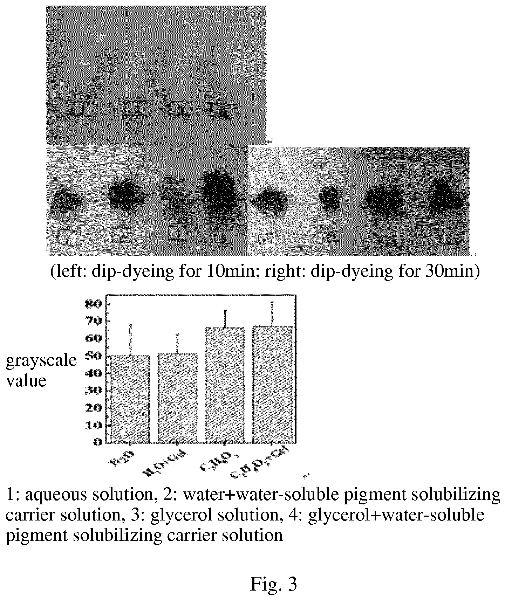

Experimental results: It can be seen from the comparison between FIG. 3 and FIG. 2 that the same composition has better dyeing effect on rabbit hair than the ink brush, and the selected glycerol-water-soluble pigment solubilizing carrier-carbon black pigment at the same time has the best dyeing effect. The reason why the dyeing effect of the ink brush hair is poorer is that the surface of the ink brush hair is chemically treated compared with the rabbit hair, so the initial dyeing effect is not reflected.

### Embodiment 4: Evaluation of blackening effect of carbon black and glycerol at different temperatures and pH

Test procedure: rabbit hair was used, 25mg hydrophilic carbon black was weighed, 5 mL deionized water was added, and the mixture was stirred overnight to fully dissolve, and a control group of aqueous solution containing black carbon was prepared, wherein the carbon black aqueous solution concentration was 5 mg/mL for use.

Different solutions (No. 1: aqueous solution (containing carbon black); No. 2: aqueous solution (containing carbon black)+1 mL polyethyleneimine-co-isopropylacrylamide (water-soluble pigment solubilizing carrier) at a concentration of 4 mg/mL (volume ratio is 4:1); No. 3: aqueous solution (containing 1 mL carbon black)+glycerol+1mL polyethyleneimine-co-isopropyl acrylamide (water-soluble pigment solubilizing carrier) (2: 2: 1)) at concentration of 4 mg/mL each was added with basic agent (such as, sodium hydroxide) to adjust the pH of the hair dye solution to obtain hair dye solution having a pH of 8 and 9. The water bath was heated to 25°C and 50°C to obtain solution of different temperatures, 10 mg dry hair was immersed for a period of time (5 min), and removed; rinsed with water, dried naturally to obtain blackened hair at\in different temperature and pH solutions.

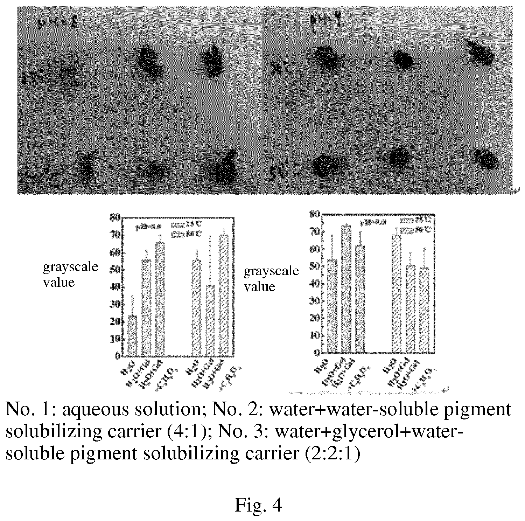

Experimental results: It can be seen from FIG. 4 that the high temperature dyeing effect is better than the low temperature dyeing effect at the measured pH, because the elevated temperature can effectively open the scaly structure of the hair surface layer, which is beneficial to the water-soluble pigment solubilizing carrier to carry the pigment into and combine with the hair. In addition, as can be seen from FIG. 4, at the measured temperature, dyeing effect at the high pH (=9) is better than that at the low pH (8), the reason is that the alkalinity of the hair dye is improved and the scaly structure of the hair surface layer can be effectively opened, which is advantageous for the water-soluble pigment solubilizing carrier to carry the pigment into and combine with the hair.

### Embodiment 5: Further evaluation of blackening effect of carbon black at different pH

Experimental Procedure: Three solutions were first prepared in accordance with Embodiments 1-3. Formulation ratio 1, 25 mg hydrophilic carbon black was weighed, 5 mL deionized water was added, and stirred overnight to fully dissolve to obtain the carbon black aqueous solution, and the concentration of carbon black aqueous solution is 5 mg/mL; at room temperature, 10 mg hair was immersed for 5 min and removed, rinsed with water, drained, and then dip-dyed in carbon black aqueous solution for a period of time, removed and naturally dried to obtain black hair blackened by carbon black aqueous solution.

Formulation ratio 2, hair blackened by carbon black and glycerol aqueous solution, 25 mg hydrophilic carbon black was weighed, 2.5 mL glycerol solvent and 2.5 mL deionized water were added and stirred overnight to fully dissolve, the concentration of carbon black was kept at 5 mg/mL, at room temperature, 10 mg hair was immersed for 5 min, and removed; rinsed with water, drained, and dried naturally to obtain the hair blackened by carbon black and glycerol aqueous solution.

Formulation ratio 3, hair blackened by carbon black and glycerol and polyethyleneimine-co-isopropyl acrylamide (water-soluble pigment solubilizing carrier) solution, 25 mg hydrophilic carbon black was weighed, 2 mL glycerol solvent, 2 mL deionized water and 1 mL polyethyleneimine-co-isopropylacrylamide (water-soluble pigment solubilizing carrier) at a concentration of 4 mg/mL were added to stir overnight to fully dissolve, the concentration of carbon black was 5 mg/mL; at room temperature, 10 mg hair was immersed in 1 mL solution for a period of time, removed, and naturally dried to obtain the hair blackened by a solution of carbon black and glycerol and polyethyleneimine-co-isopropylacrylamide (water-soluble pigment solubilizing carrier).

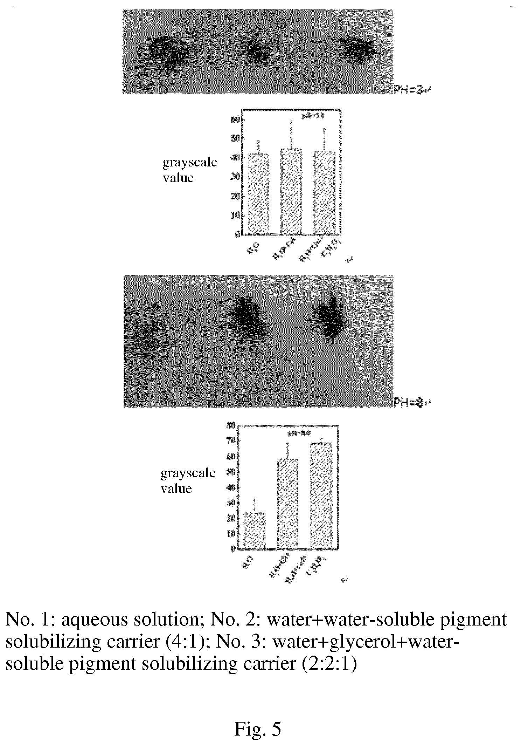

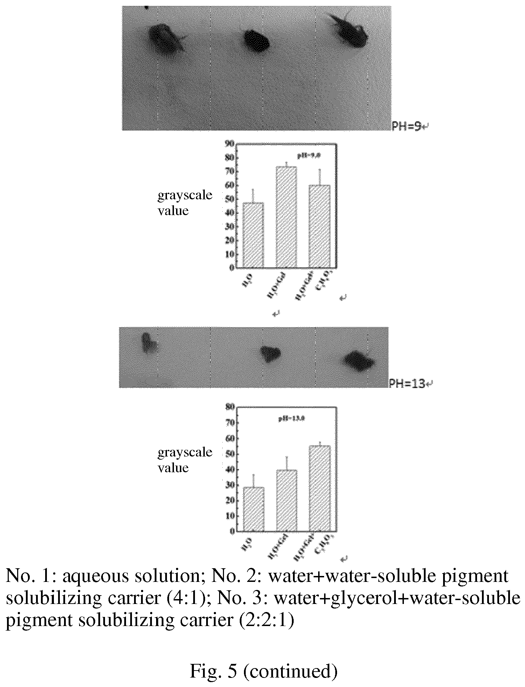

Experimental results: As can be seen from FIG. 5, in the hair dye at the determined ratio, all dyeing effects was the higher the basic condition (the higher the pH value), the better the dyeing effect, and the formulation ratio 3 (water-alcohol-water-soluble pigment solubilizing carrier) has the best hair dyeing effect. However, when the pH reached 13, the hair quality of the hair received a slight degree of damage, and the apparent appearance was agglomeration of the hair. It can be seen that high pH is good for dyeing, but not the higher the better.

### Embodiment 6: Effect of different dyeing time and pH on carbon black dyeing

Experimental procedure: According to Embodiments 1-3, different solutions (No. 1: aqueous solution; No. 2: water+polyethyleneimine-co-isopropyl acrylamide (water-soluble pigment solubilizing carrier) (4:1, volume ratio, the same below); No. 3: water+glycerol+polyethyleneimine-co-isopropyl acrylamide (water-soluble pigment solubilizing carrier) (2:2:1)) each was added with an basic agent (for example, sodium hydroxide) to adjust the pH of the solution to obtain a hair dye solution having a pH of 9 and 13. At room temperature, 10 mg rabbit hair was immersed in the hair dye solution with pH 9 and 13, and the different hair dyeing time was controlled at 10 min or 30 min, respectively, and the hair was removed and naturally dried to obtain hair dyeing results of different pH values and different time..

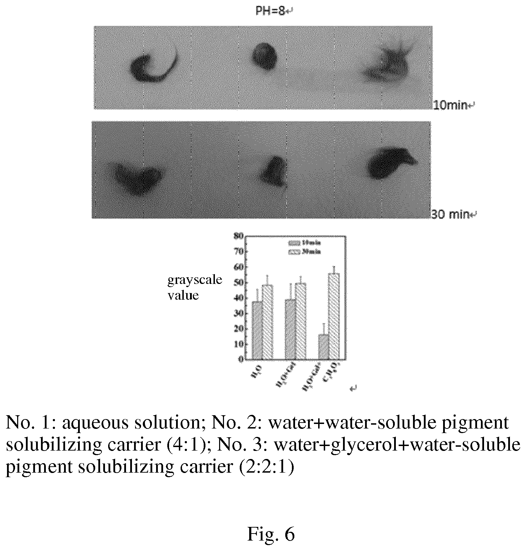

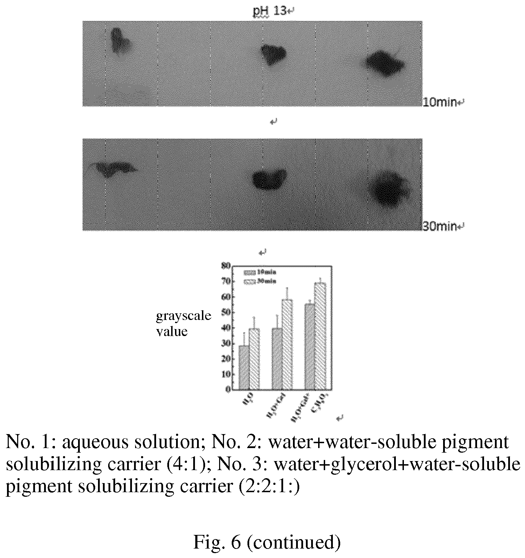

Experimental results: It can be seen from FIG. 6 that under weak basic conditions (pH 9), the dyeing effect of each group becomes better with the prolongation of time (the gray value increases), moreover, the water-glycerol-water-soluble pigment solubilizing carrier has the best effect. However, when the alkalinity of the hair dye solution is increased to 13, although the effect is good in a short period of time, the hair is still damaged to some extent, and the appearance is that the hair is agglomerated, softened and easily broken. Moreover, the damage is further exacerbated over time. It can be seen that the effect of improving hair dyeing is not as high as possible. In addition, the damage is more pronounced in the water-insoluble pigment solubilizing carrier group. It can be seen that our water-soluble pigment solubilizing carrier has a certain protective effect on hair.

### Embodiment 7: Comparison of microstructures of hair (human and rabbit hair) before and after hair dyeing

Experimental procedure: According to Embodiments 1-3, hair was blackeded by carbon black and glycerol and polyethyleneimine-co-isopropylacrylamide (water-soluble pigment solubilizing carrier) solution. 25 mg of hydrophilic carbon was weighed, 2 mL glycerol solvent, 2 mL deionized water and 1 mL of 4 mg/mL polyethyleneimine-co-isopropyl acrylamide (water-soluble pigment solubilizing carrier) were added and stirred overnight to fully dissolve. The concentration of carbon black is 5 mg/mL, and 10 mg of human hair was immersed in 1 mL solution at room temperature for about 5 min, removed, and naturally dried to obtain the hair blackened by a glycerin water-soluble pigment solubilizing carrier solution. At the same time, undyed rabbit hair from the same source was used as a control for microscopic examination (TEM).

According to Embodiment 1-3, hair was blackened by carbon black-glycerol-polyethyleneimine-co-isopropylacrylamide (water-soluble pigment solubilizing carrier) solution. 25 mg hydrophilic carbon black was weighed, 2 mL glycerol solvent, 2 mL deionized water and 1 mL polyethyleneimine-co-isopropylacrylamide (water-soluble pigment solubilizing carrier) at a concentration of 4 mg/mL were added and stirred overnight to fully dissolve, The concentration of carbon black was 5 mg/mL. 10 mg hair was immersed in 1 mL solution at room temperature for 5 min, removed, and naturally dried to obtain the hair blackened by a solution of glycerol and polyethyleneimine-co-isopropylacrylamide (water-soluble pigment solubilizing carrier). At the same time, the hair of the dyed person from the same source was selected as a control to perform microscopic examination (TEM).

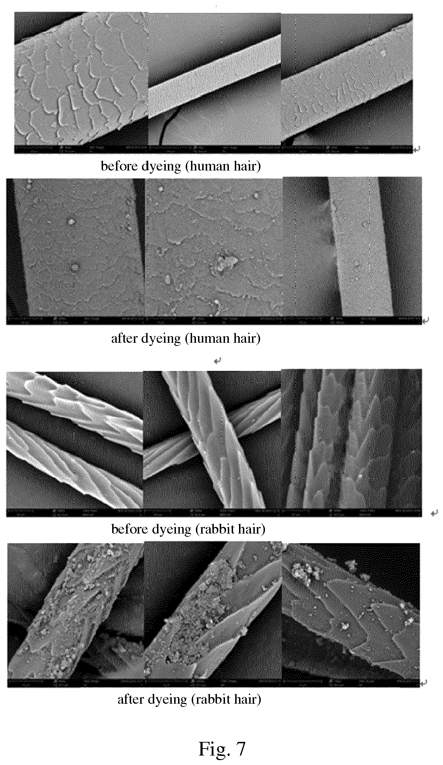

Experimental results: As can be seen from FIG. 7, when comparing the hair before and after dyeing in the microscope, whether it is human hair (the upper two groups) or rabbit hair (the lower two groups), the scales of the dyed hair are obviously enriched with a lot dense particles, showing that our water-soluble pigment solubilizing carrier can significantly promote the enrichment of the pigment in the scale structure of the hair surface and improve the dyeing effect.

### Embodiment 8. Effect of water-soluble pigment solubilizing carrier and different pigments on white hair

Experimental procedure: selection of rabbit hair, polyethyleneimine-co-isopropylacrylamide (water-soluble pigment solubilizing carrier), glycerol, pigment (black pigment, obtained by basic red : basic yellow : basic blue =0.01-0.3:0.7-5:0.4-4 (weight ratio); a natural plant pigment obtained by carmine : lemon yellow : bright blue=0.1-3:0.03-2:0.1-10 (weight ratio). 20 mg of each pigment was added, and 4 ml water was added to prepare a solution having a concentration of 5 mg/ml. The polyethyleneimine-co-isopropylacrylamide (water-soluble pigment solubilizing carrier) concentration was 50 mg/mL. 12 tubes were divided into 2 groups (including polyethyleneimine-co-isopropyl acrylamide (water-soluble pigment solubilizing carrier) and no polyethyleneimine-co-isopropyl acrylamide (water-soluble pigment solubilizing carrier)), each group of 6 tubes. In a group of tubes without polyethyleneimine-co-isopropyl acrylamide (water-soluble pigment solubilizing carrier), 0.1 ml pigment and 1.9 ml water were added; in the group of tubes containing the carriers, 1.86 ml water+0.04 ml water-soluble pigment solubilizing carrier+0.1 ml pigment was added, and the total volume was 2 ml, and the mixture was allowed to stand for 15 min. At room temperature, an appropriate amount of white rabbit hair was added to 12 tubes, dyed for 5 min, removed, and washed twice in water to observe the color of rabbit hair

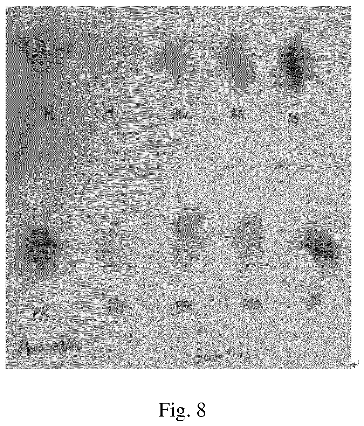

Experimental results: As can be seen from FIG. 8, the color of the rabbit hair in the No. 1 tube is lighter than that of the No. 2 tube (the tube group containing the carrier), and the rabbit hair in the No. 3 and No. 4 tubes (the tube group without polyethyleneimine-co-isopropylacrylamide (water-soluble pigment solubilizing carrier) has almost no color. Therefore, the experimental results show that polyethyleneimine-co-isopropylacrylamide (water-soluble pigment solubilizing carrier) can significantly enhance the dyeing effect of red, blue, yellow and black carbon black pigments (dyeing effects scores are 4, 4, 4 and 4 respectively).

### Embodiment 9. Effect of different water-soluble pigment solubilizing carrier-loaded black pigment on white hair

Experimental procedure: rabbit hair, different water-soluble pigment solubilizing carriers (sodium alginate, chitosan, polyethyleneimine, polylysine, sodium alginate-polyethyleneimine), and natural plant pigments obatined by carmine : lemon yellow : bright blue = 0.1-3:0.03-2:0.1-10 (weight ratio) were selected. 20 mg of each pigment was added, and 4 ml water was added to prepare a solution having a concentration of 5 mg/ml. The water-soluble pigment solubilizing carrier (sodium alginate, chitosan, polyethyleneimine, polylysine, sodium alginate-polyethyleneimine) has a concentration of 50 mg/mL. With 6 tubes, 1.86 ml water+0.04 ml water-soluble pigment solubilizing carrier+0.1ml pigment was added to the tube group containing carrier, with a total volume of 2 ml, mixed and let stand for 15 min. At room temperature, an appropriate amount of white rabbit hair was added to 6 tubes, dyed for 5 min, removed, and washed twice in water to observe the color of the rabbit hair.

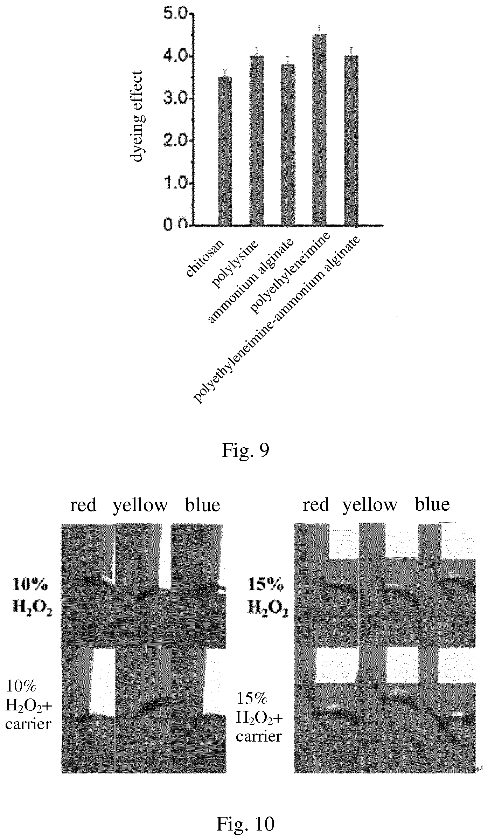

Experimental results: As can be seen from FIG. 9, the rabbit hairs in each group were well dyed with black color. Therefore, the experimental results show that different water-soluble pigment solubilizing carriers can significantly enhance the dyeing effect of red, blue, yellow and black carbon black pigments.

### Embodiment 10. simulation of commercially available pigments (red, blue, yellow) dyeing processes to black human hair by water-soluble pigment solubilizing carrier and effect examination

Experimental materials: Human hair, pigment (basic red, basic yellow, basic blue), and H₂O₂ were selected. At the same time, the preparation of polyethyleneimine-co-isopropylacrylamide (water-soluble pigment solubilizing carrier) dye: 50 mg/ml water-soluble pigment solubilizing carrier mother liquor was prepared, with pigment mother liquor concentration of 20 mg/ml, and 10%, 15% H₂O₂ solution was prepared. First, black hair was pretreated with 50 mL H₂O₂ (volume ratio less than 10%) for 1 hour to simulate the commercial dyeing process. After adding an appropriate amount of H₂O₂ solution pigment solution to the two solutions, it was allowed to stand for 1H. 12 tubes were numbered a 10% group (R red, Y yellow, BLU blue, RP red+carrier, YP yellow+carrier, BLUP blue+carrier), and a 15% group (R, Y, BLU, RP, YP, BLUP). Here, R, Y, and BLU represent red, yellow, and blue, respectively. At room temperature, an appropriate amount of hair was added to the 10% group and the 15% group, respectively, dyed for 10 min, washed with water, and photographed.

Experimental results: As can be seen from FIG. 10, the water-soluble pigment solubilizing carrier can quickly transfer the three colors of red, blue and black to the black human hair, and the dyeing effect will not change much with the addition of hydrogen peroxide (the dyeing effect score is 3.5).

### Embodiment 11. Dyeing of human black hair by water-soluble pigment solubilizing carrier (red, blue, yellow)

Experimental materials: Human hair, pigment (carmine, lemon yellow, bright blue) and H₂O₂ were selected. At the same time, polyethyleneimine-co-isopropylacrylamide (water-soluble pigment solubilizing carrier) is prepared: 50 mg/ml polyethyleneimine-co-isopropylacrylamide (water-soluble pigment solubilizing carrier) mother liquor was prepared with the pigment mother liquor concentration of 20 mg/ml, and 10%, 15% H₂O₂ solution was prepared, and about 10% (by volume) was added to the prepared water-soluble pigment solubilizing carrier. 12 tubes were numbered a 10% group (R: red; Y: yellow; BLU: blue; RP: red+carrier; YP: yellow+carrier; BLUP: blue+carrier, (the same below), and a 15% group (R, Y, BLU, RP, YP, BLUP) respectively, an appropriate amount of hair was added to the 10% group and the 15% group, dyed for 10 min, washed with water, and photographed.

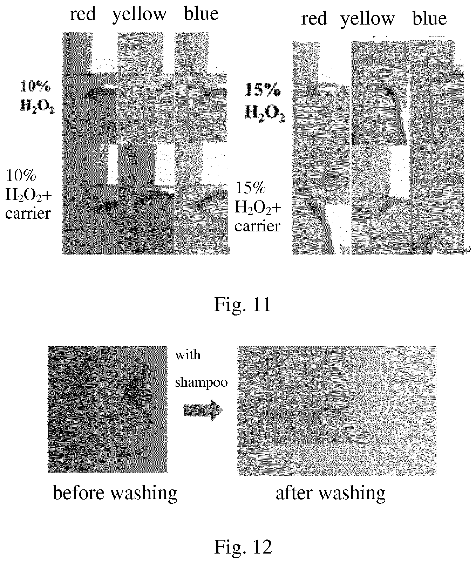

Experimental results: As can be seen from FIG. 11, the water-soluble pigment solubilizing carrier can quickly transfer the red, yellow and blue colors to the human black hair, and the dyeing effect will not change much with the addition of hydrogen peroxide. Compared with traditional hair dyeing, this result has the following three advantages: 1. the water-soluble pigment solubilizing carrier can significantly accelerate the dyeing of the pigment, and the time is about 10 min; 2, the pigment prepared by the water-soluble pigment solubilizing carrier can increase the adhesion of color; 3, the addition of a small amount of oxidant has little effect on the dyeing effect (the dyeing effect score is 3.5).

Embodiment 12. The hair was washed with shampoo, and the ability of the water-soluble pigment solubilizing carrier to color the pigment was observed.

Experimental procedure: According to the embodiment 10, basic red yellow blue, and polyethyleneimine-co-isopropylacrylamide (water-soluble pigment solubilizing carrier) were prepared: 50 mg/ml polyethyleneimine-co-isopropylacrylamide mother liquor (water-soluble pigment solubilizing carrier) was prepared, with pigment mother liquor concentration of 20 mg/ml, and 10%, 15% H₂O₂ solution was prepared. An appropriate amount of H₂O₂ solution (volume ratio less than 10%) was added to the two solutions. After the pigment solution was prepared, it can be allowed to stand for 1h. 12 tubes were numbered a 10% group (R, Y, BLU, RP, YP, BLUP, same as embodiment 11) and a 15% group (R, Y, BLU, RP, YP, BLUP, same as embodiment 11) respectively. Appropriate rabbit hair was added to the 10% group and the 15% group, dyed for 10 min, and dried. Then for a sample of red dyed hair, the dyed hair was hand-washed artificially with commercially available shampoo (rejoice) for 1 min, washed with water, and photographed In this test, we also used a dyed group of non-water soluble pigment solubilizing carrier for comparison.

Experimental results: As can be seen from FIG. 12, the water-soluble pigment solubilizing carrier can quickly dye the red color on, and the pigment dyed does not fall off substantially with washing of the shampoo. It illustrates that the solubilizing carrier can not only be dyed, but also enhance the adhesion of the pigment (the dyeing effect score is 4.5).

Embodiment 13. The effect of water-soluble pigment-soluble carrier on the hair quality after dyeing was observed.

Experimental procedure: hair blackened by solution of carbon black-glycerol-polyethyleneimine-co-isopropylacrylamide (water-soluble pigment solubilizing carrier). 25 mg hydrophilic carbon black was weighed, and 2 mL glycerol solvent, 2 mL deionized water and 1 mL polyethyleneimine-co-isopropylacrylamide (water-soluble pigment solubilizing carrier) were added and stirred overnight to fully dissolve, with carbon black concentration of 5 mg/mL. At room temperature, 10 mg hair was immersed for 5 min and removed, naturally dried to obtain the hair blackened by the solution of glycerol-polyethyleneimine-co-isopropylacrylamide (water-soluble pigment solubilizing carrier). At the same time, dyed human hair from the same source was used as a control, and YC020B electronic single yarn strength machine was used to detect hair before and after dyeing, high temperature hair dyeing group (50 degrees), basic hair dyeing group (pH=10), non-dyed hair and chemical hair dyed hair for tensile test.

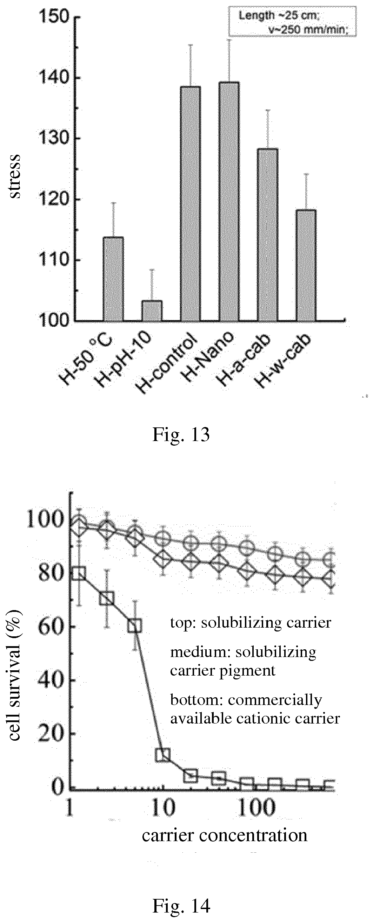

Experimental results: As can be seen from FIG. 13, as shown in the above-mentioned embodiments, the hair was damaged more under the conditions of too high temperature and basic, and the hair was not suitable for stretching. In contrast, our water-soluble pigment solubilizing carrier group and the non-dyed hair group have the strongest tensile strength, and the chemical hair dyeing group causes hair quality to deteriorate. Therefore, the dyeing conditions and process of our solubilizing carrier group have protective effect on hair.

Embodiment 14. The biocompatibility of water soluble solubilizing carrier, pigment and commercially available cationic formulation was observed.

According to embodiments 1-3, water-soluble pigment solubilizing carrier dye was prepared, while cationic carrier (sodium lauryl sulfate) was used. First, human epithelial-derived cells (293T) were cultured, which was divided into three groups corresponding to a polyethyleneimine-co-isopropylacrylamide (water-soluble pigment solubilizing carrier) group, a polyethyleneimine-co-isopropylacrylamide (a water-soluble pigment solubilizing carrier) dye, and a commercially available cationic carrier group; the cells were cultured with commercially available cell culture medium (160DM) for 24 hours to obtain cells in the logarithmic growth phase; at this time, the same concentration gradient of polyethyleneimine-co-isopropylacrylamide (water-soluble pigment solubilizing carrier), polyethyleneimine-co-isopropylacrylamide (a water-soluble pigment solubilizing carrier)-pigment, and commercially available cationic carrier were added to the culture dishes of the three groups of cells; after 24 hours of culture, the CCK-8 test kit was used to examine the killing ability of the three groups of systems to the cells.

Experimental results: As can be seen from FIG. 14, the cells were hardly damaged by the action of the water-soluble pigment solubilizing carrier group and the water-soluble pigment solubilizing carrier-pigment group, whereas in the commercially available cationic carrier group, the cell death was rapid (see FIG. 14). Therefore, the hair dye of the water-soluble pigment solubilizing carrier has no damage to hair and human cells, and has good biocompatibility.

### Embodiment 15. Hair dyeing effect of water-soluble pigment solubilizing carrier and commercially available hair dye

The water-soluble pigment solubilizing carrier was compared with commercially available black paste (purchased by Guangzhou BoRou Cosmetics Co., Ltd., product barcode: 4987205051166):

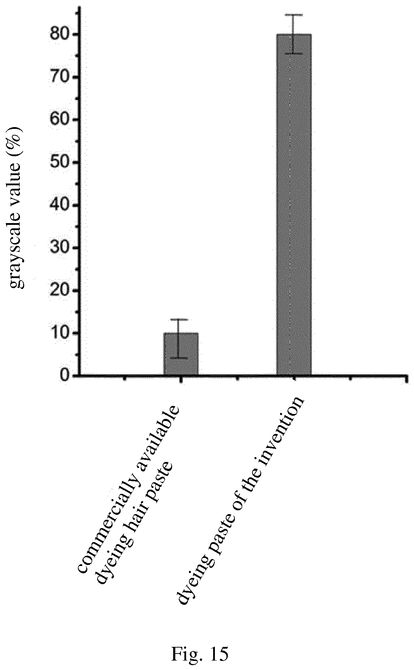

A solution containing carrier polyethyleneimine-co-isopropylacrylamide (water-soluble pigment solubilizing carrier) was prepared at a concentration of 10-50 mg/ml, numbered as A; an appropriate amount of commercially available black paste dye was numbered as B, A and B were placed in two holes in the palette respectively; an appropriate amount of yak hair were put and dyed for 15 min, and washed by water to observe the dyeing effect. The result is shown in FIG. 15. Results: B appeared light gray and the color of A is deeper.

Conclusions: Experiments have shown that water-soluble pigment solubilizing carriers can be quickly colored, and B takes longer time, usually 1 hour.

### Embodiment 16. Formulation and hair dyeing effect of paste based on water-soluble pigment solubilizing carrier

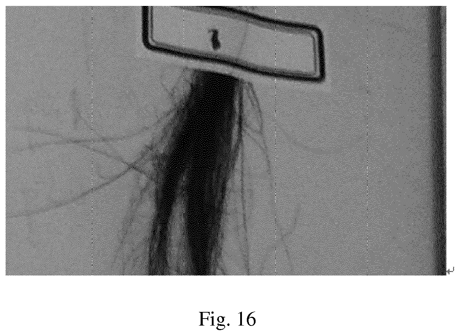

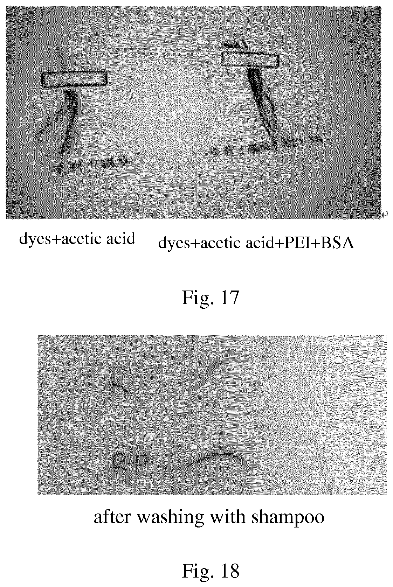

(I) Improved effects of water-soluble pigment solubilizing carrier in commercially available black paste: the specific embodiment is as follows: the carrier polyethyleneimine-co-isopropylacrylamide (water-soluble pigment solubilizing carrier) was prepared at a concentration of 10-50 mg/ml; an appropriate amount of black paste dye (conventional mixing of various ingredients) was placed in two holes in the palette, numbered A and B respectively: A. black paste dye, B. black paste dye+400µl polyethyleneimine-co-isopropylacrylamide (water-soluble pigment solubilizing carrier); an appropriate amount of yak hair was put, dyed, and washed by water to observe the dyeing effect. The result is shown in FIG. 16. Results: it appeared light gray, and the color of the carrier added was deeper. The conclusion was that the water-soluble pigment solubilizing carrier had a certain hair dyeing effect (please give each group a score). Conclusion: the rapid experiment proves that the water-soluble pigment solubilizing carrier can not only enhance the dyeing of the formulated dye quickly, but also have rapid enhancement effect (the dyeing effect score is 4) within 10 min for the dyeing paste that is commercially available and takes 1 hour of dyeing time.
(2) The basic red, yellow and blue melanin hair dyeing paste formulation and hair dyeing effect thereof based on the above-mentioned three primary colors red, green and blue. Specific embodiment: the water-soluble pigment solubilizing carrier mother liquor was prepared at a concentration of 50-100 mg/ml, and the concentration of BSA mother liquor was 10-30 mg/ml glacial acetic acid (1:5 dilution); experiment 1: yak hair was treated with a mixed liquid composed of 1 ml polyethyleneimine-co-isopropylacrylamide (water-soluble pigment solubilizing carrier)+2 ml BSA for 5 min, and removed after 5 min, with the residual liquid on the surface removed; the creamy paste (the above-mentioned embodiment) made of black dye (large bottle) was evenly spread on the surface of the hair and dyed for 10 min; after 10 min, the hair was removed and washed off the paste on the surface. The result is shown in FIG. 17. Results: the yak hair appeared black (right) (the dyeing effect score was 0.5 and 4 points respectively)). Conclusion: Based on the three primary colors red, green and blue and the water-soluble pigment solubilizing carrier, we can formulate out black dyeing paste, and the dyeing paste can quickly and easily dye white hair.
(3) Natural color pigmented hair dyeing paste based on water-soluble pigment solubilizing carrier. The specific embodiment is as follows: (1) tinting: 125.2mg the red dye (R) was added with 10.841 mL water to obtain the mother liquor for use, and the carrier polyethyleneimine-co-isopropylacrylamide (water-soluble pigment solubilizing carrier) concentration is prepared at 50 mg/ml; mixed liquid I: R:P800=1.4 ml: 0.2 ml; preparation of one dose of mixture I, the total volume was 18.55 ml; 3.8 g petrolatum, 3.0 g sodium alginate, and 1.0 g PEG were added into a 50 ml beaker, placed in a water bath at 60°C, heated and stirred continuously until melting, mixed dye solution was added under constant stirring, and continued to stir for about 5 min after appearing semi-solid paste, removed and stirred continuously until condensation; an appropriate amount of yak hair was put in a culture dish, coated with the paste dye and dyed for 15 min. The result is shown in FIG. 18. Conclusion: Based on the water-soluble pigment solubilizing carrier and red dye, red dyeing paste can be blended out, and can allow quickly and easily dyed red (the dyeing effect score is 1.5 and 4 respectively).

The preferred embodiments of the present invention have been specifically described above, but the present invention is not limited to the embodiments, and those skilled in the art can make various equivalent variations or alternatives without departing from the spirit of the present invention. The equivalent variations or alternatives are intended to be included within the scope of the appended claims.

## Claims

1. A composition, the composition comprising:
pigments, 1-40% by mass in the composition;
water-soluble pigment solubilizing carriers, 1-50% by mass in the composition; and
optional additive.

2. The composition according to claim 1, wherein the water-soluble pigment solubilizing carrier is selected from polymer, copolymer or inorganic material.

3. The composition according to claim 2, wherein the polymer is a polymer containing carboxyl group, amino group, cationic group and anionic group, the inorganic material is inorganic molecule containing silicon and oxygen elements.

4. The composition according to claim 3, wherein the polymer is a polymer selected from polyethyleneimine, polylysine, chitosan, sodium alginate, and polyacrylic acid; and these polymers and copolymers thereof, as well as hybrids and composite materials of the copolymer of the polymer and inorganic material selected from silicon or iron.

5. The composition according to claim 4, wherein the polymer is a polymer selected from polyethyleneimine, polylysine, chitosan, sodium alginate, polyacrylic acid, and the copolymer of the above-mentioned polymer linked with poly-isopropylacrylamide or polymethylmethacrylate.

6. The composition according to claim 2, wherein the copolymer is a copolymer selected from polyethyleneimine-co-isopropylacrylamide, sodium alginate-co-polyethyleneimine, polyethyleneimine-co-isopropylacrylamide, chitosan-co-isopropylacrylamide, chitosan chain, and a nanocomposite system formed by non-chemical crosslinking.

7. The composition according to any of claim 3-6, wherein the water-soluble pigment solubilizing carrier comprises forms of gel, latex, inorganic-organic hybrid composite, and microsphere or nanogel formed by self-assembly.

8. The composition according to any of claim 1-7, wherein the pigment is a pigment molecule selected from basic red, basic yellow, and basic blue, and a black pigment obtained by formulating the pigment molecules in a certain ratio.

9. The composition according to claim 8, wherein the black pigment is obtained by formulating according to the basic red: basic yellow: basic blue of 0.01-0.3:0.7-5:0.4-4 by weight ratio.

10. The composition according to claim any of 1-7, wherein the pigment is a natural plant pigment molecule selected from carmine, lemon yellow, and bright blue, and a natural plant black pigment obtained by formulating the natural plant pigment molecules in a certain ratio.

11. The composition according to claim 10, wherein the pigment is carbon black, graphene or mixture thereof, wherein the natural plant black pigment is obtained by formulating according to carmine: lemon yellow: bright blue of 0.1-3:0.03-2:0.1-10 by weight ratio.

12. The composition according to any of claim 1-7, wherein the pigment is carbon black, graphene or mixture thereof.

13. The composition according to any of claim 1-12, wherein the water-soluble pigment solubilizing carrier is a particle range from 0.1nm to 5000 nm.

14. The composition according to any of claim 13, wherein, the water-soluble pigment solubilizing carrier is a particle range from 0.1nm to 500 nm.

15. The composition according to any of claim 1-14, wherein the additive is/are selected from a derivative of glycerol/or salt or ester thereof, or a derivative of tridecyl polyether-2/or salt or ester thereof, and pentasodium pentoxide/or Tetrasodium pyrophosphate/or sodium stannate.

16. The composition according to any of claim 1-15, wherein the pH of the composition is adjusted to less than about 13 by the basic agent, preferably 6<pH<13, more preferably 9<pH<13, and most preferably 8<pH<10.

17. The composition according to claim 16, wherein the basic agent is alkanolamine and inorganic compound.

18. The composition according to claim 17, wherein the alkanolamine is a derivative of monoethanolamine, polyethanolamine or propylene glycol.

19. The composition according to claim 17, wherein the inorganic compound is sodium hydroxide, sodium carbonate, sodium bicarbonate, calcium carbonate or calcium bicarbonate.

20. The composition according to claim 3, the copolymer is prepared by the following method:
the free radical initiator initiates the first monomer to react with the second monomer both of which containing amino groups to form a copolymer by free radical graft copolymerization in an aqueous solution and at temperature of 60-100°C.

21. The composition according to any of claim 1-20, the composition is solution, paste or emulsion.

22. A method of applying the composition according to any of claims 1 to 21 in dyeing hair, comprising the steps of:
coating semi-wet or dried hair with the composition at 25°C -50°C, rinsing with water after 3-15 min, preferably 3-10 min, and more preferably 5-10 min, drying naturally so as to complete the hair dyeing.

23. The method according to claim 22, the method comprises pretreating the hair with pretreatment composition before dyeing hair for less than about 10 min, preferably 3-10 min.

24. The method according to claim 23, wherein the pretreatment composition is an aqueous solution of 2-mercapto-ethanol and lysine homopolymer with the weight ratio of 2-mercapto-ethanol and lysine being 1:3-1:10, and the aqueous solution concentration of the composition is 0.1-10 mg/mL.

25. Use of the composition according to any of claims 1 to 21 in the preparation of hair dye.

26. A method for preparing the composition according to any of claims 1 to 21, the method comprises the step of mixing all the components.
